# EUROPEAN PATENT SPECIFICATION

(11) **EP 2 184 605 B1**
(45) Date of publication and mention of the grant of the patent: **17.02.2016**
(21) Application number: 08805353.3
(22) Date of filing: 28.07.2008
(51) Int. Cl.: G01N 33/53, G01N 33/569

(54) **DUAL-RECOGNITION IMMUNOASSAY FOR THE DETECTION OF ANTIBODIES**
IMMUNASSAY MIT DOPPELERFASSUNG ZUR DETEKTION VON ANTIKÖRPERN
IMMUNOESSAI A DOUBLE RECONNAISSANCE POUR LA DETECTION D'ANTICORPS

(30) Priority: 27.07.2007 ES 200702152
(43) Date of publication of application: 12.05.2010
(73) Proprietor: INMUNOLOGIA Y GENETICA APLICADA, S.A., 28037 Madrid (ES)
(72) Inventor: VELA OLMO, Carmen, E-28037 Madrid (ES); VENTEO MORENO, Angel Antonio, E-28037 Madrid (ES); SANZ FERNÁNDEZ, Antonio José, E-28037 Madrid (ES)
(74) Representative: ABG Patentes, S.L.
(86) International application number: PCT/ES2008/000524
(87) International publication number: WO 2009/027550

(56) References cited:
- WO-A1-91/10136
- WO-A2-2006/091824
- WO-A2-2006/093797
- WO-A2-2007/052238
- FEHRSEN ET AL: "Serogroup-reactive and type-specific detection of bluetongue virus antibodies using chicken scFvs in inhibition ELISAs" JOURNAL OF VIROLOGICAL METHODS, ELSEVIER BV, NL LNKD- DOI:10.1016/J.JVIROMET.2005.04.015, vol. 129, no. 1, 1 October 2005 (2005-10-01), pages 31-39, XP005050667 ISSN: 0166-0934
- LUNT R A ET AL: "EVALUATION OF A MONOCLONAL ANTIBODY BLOCKING ELISA FOR THE DETECTION OF GROUP-SPECIFIC ANTIBODIES TO BLUETONGUE VIRUS IN EXPERIMENTAL AND FIELD SERA" 1988, JOURNAL OF GENERAL VIROLOGY, VOL. 69, NR. 11, PAGE(S) 2729-2740 , XP002590777 ISSN: 0022-1317 * page 2730 - page 2731 *
- SORENSEN K.J. ET AL.: 'Blocking ELISA's for the distinction between antibodies against european and american strains of porcine reproductive and respiratory syndrome virus' VETERINARY MICROBIOLOGY vol. 60, no. 2-4, 28 February 1998, pages 169 - 177, XP008129824

## Description

### Field of the Invention

The invention generally relates to a dual-recognition immunoassay for the detection of antibodies in a sample with high specificity and sensitivity. The invention also relates to a kit comprising the components necessary for performing said immunoassay.

### Background of the Invention

An immunoassay is an assay based on the antigen-antibody binding specificity which allows detecting and optionally quantifying antigens or antibodies. Immunoassays can be classified as immunoassays using non-labeled reagents (antigens or antibodies) and immunoassays using labeled reagents wherein the reagent (antigen or antibody) is labeled with a marker, such as a radioactive isotope, an enzyme, a fluorophore or a substance involved in a (chemo) luminescent reaction, giving rise to so-called radioimmunoassays (e.g., RIA, IRMA), enzyme immunoassays (e.g., EMIT, CEDIA, ELISA), fluoroimmunoassays (e.g., FPIA, SLFIA, FETI, DELFIA) or luminescent immunoassays, respectively.

Included among the immunoassays that use labeled reagents are homogeneous immunoassays and heterogeneous immunoassays; the latter comprise performing one or more steps of separating the antigen or antibody not bound to the other member of the binding pair (antibody or antigen) which will generally be bound to a solid phase or support, for example, microplates, magnetic particles, nitrocellulose membranes, etc. Furthermore, depending on the design of the assay, heterogeneous immunoassays can be competitive or non-competitive, depending on if the labeled reagent (antigen or antibody) is added in a limited amount or in excess, respectively.

Immunoassays allow identifying antigens (direct techniques) or antibodies against an antigen (indirect techniques). These indirect techniques include indirect immunoassays and blocking or competitive immunoassays. Despite the fact that indirect immunoassays have high sensitivity, they have specificity problems because of the false positives obtained, usually due to unspecific core problems. Furthermore, they do not allow detecting IgMs because anti-IgG antibodies are typically used, so they do not detect positive animals until 2-3 weeks post-infection or post-vaccination. Blocking or competitive immunoassays are generally more specific than indirect immunoassays but less sensitive.

Due to the specificity and sensitivity of immunoassays, a number of immunoassays have been developed for various applications, both for their use in the analysis of environmental samples or of foods, and for diagnostic purposes. By way of illustration, Blue Tongue and Porcine Reproductive and Respiratory Syndrome (PRRS) are included among the infections caused by animal viruses which can be diagnosed by means of immunoassays.

Blue Tongue is a disease caused by the Blue Tongue Virus (BTV), an arbovirus (virus transmitted by arthropods) which naturally infects domestic and wild ruminants, camelids and other herbivores, such as elephants, although the disease concerns sheep almost exclusively, generating acute or sub-acute clinical courses. In cows and goats, clinical disease is rare and when it presents, it is much milder than in sheep. The concerned sheep can die after an acute or chronic disease, or it can recover with weight loss and/or wool breaks. BTV is the prototypical virus of the Orbivirus genus (*Reoviridae* family). The BTV genome includes 10 different segments of double stranded RNA (dsRNA) encoding 7 structural proteins (VP1 to VP7) and 4 non-structural proteins (NS1, NS2, NS3 and NS3a). The inner core of BTV is formed by 2 majority proteins (VP3 and VP7) and by 3 minority proteins (VP1, VP4 and VP6) and is surrounded by an outer capsid formed by VP2 and VP5 (WP 2008/030282). There are currently 24 recognized BTV serotypes. The recommended tests for the detection of specific serogroups and antibodies of BTV include agar gel immunodiffusion and a competitive ELISA, preferably the latter due to its better accuracy and adaptation to fast conventional laboratory tests and reading technology. The test recommended for detecting serotype-antibodies specific to BTV is the neutralization test.

The Porcine Reproductive and Respiratory Syndrome Virus (PRRSV) is an arterivirus that causes the Porcine Reproductive and Respiratory Syndrome (PRRS). Said virus can cause important reproductive problems in female adult pigs that can result in miscarriages as well as respiratory problems, among others, in newborn piglets or in developing pigs. European patent EP 952219 B1 describes a method for the diagnosis of PRRSV which comprises contacting a biological sample from an animal with a recombinant fusion protein comprising the amino acid sequence of the nucleocapsid (N) protein of PRRSV fused to a fragment of the protein of gene 10 of phage T7.

### Summary of the Invention

It has now surprisingly been observed that the addition of a conjugate comprising an antigen and a marker to an antigen-antibody complex increases the specificity and sensitivity of an immunoassay for the detection of antibodies specific to said antigen.

The immunoassay provided by this invention is referred to as "dual-recognition immunoassay" because it comprises the recognition and binding of two units of the target antigen to the antibody specific to said target antigen, one of them forming part of a conjugate comprising said target antigen and a marker . Said immunoassay has been validated in reference sera for the 24 recognized serotypes of Blue Tongue Virus (BTV) (Example 1) as well as in the detection of antibodies specific to the Porcine Reproductive and Respiratory Syndrome Virus (PRRSV) (Example 3) and it has a high specificity and sensitivity, as is clearly shown in Example 2.

Said dual-recognition immunoassay can be used to detect and, if desired, to quantify antibodies specific to antigens of pathogenic organisms in a test sample from a subject , and to thus diagnose the infection caused by an organism, such as a pathogenic organism in a subject that can be infected by said Fehrsen evaluates two BTV serotype 10-specific single-chain Fv chicken antibody fragments (scFvs) in a competitive ELISA for detection of BTV antibodies (Fehrsen J et al. 2005 J Virol Methods 129: 31-39). Lunt describes a blocking ELISA incorporating monoclonal antibody 20E9B7G2 for BTV antibody detection (Lunt RA et al. 1988 J Gen Virol 69: 2729-2740). International patent application WO 91/10136 A1 describes a method of diagnosing bluetongue infection in an animal that comprises contacting an antibody-containing sample with an antigen reagent comprising at least one epitope of bluetongue protein VP6 and detecting binding of the antibody to said antigen reagent. International patent application WO 2007/052238 A2 describes a chimeric antigen including an orbivirus Vp7 polypeptide, as well as a method of inducing an immune response in a subject that comprises administering the chimeric antigen to a subject. International patent application WO 2006/093797 A2 describes methods, devices, and kits for the detection of PRRS virus based on an agent that binds the nucleocapsid (N) protein of PRRSV. International patent application WO 2006/091824 A2 describes compositions and methods for the detection and quantification of PRRSV antibodies based on polypeptides.
, wherein the target antigen is an antigen of a virus belonging to the *Arteriviridae* or *Reoviridae* family
wherein the target antigen is an antigen of a virus belonging to the *Arteriviridae* or *Reoviridae* family, organism, preferably in a human being.

Therefore, in one aspect the invention relates to an immunoassay to detect an antibody specific to a target antigen in a sample which comprises contacting said target antigen with a sample suspected of containing said antibody specific to said target antigen under conditions allowing the formation of an antigen-antibody complex, adding a conjugate comprising said target antigen and a marker under conditions allowing the formation of an antigen-antibody-antigen/marker complex, and detecting said antigen-antibody-antigen/marker complex . In a particular embodiment, said antibody is an antibody specific to BTV, whereas in another particular embodiment, said antibody is an antibody specific to PRRSV.

In another aspect, the invention relates to a kit comprising a support comprising a target antigen and a conjugate comprising said target antigen and a marker . In a particular embodiment, said target antigen is a BTV antigen, whereas in another particular embodiment, said target antigen is a PRRSV antigen.

### Detailed Description of the Invention

In one aspect, the invention relates to an immunoassay, hereinafter immunoassay of the invention, to detect an antibody specific to a target antigen in a sample which comprises:
a) contacting said target antigen with a sample suspected of containing said antibody specific to said target antigen under conditions allowing the formation of an antigen-antibody complex;
b) adding a conjugate comprising said target antigen and a marker under conditions allowing the formation of an , wherein the target antigen is an antigen of a virus belonging to the *Arteriviridae* or *Reoviridae* family
   , wherein the target antigen is an antigen of a virus belonging to the *Arteriviridae* or *Reoviridae* family antigen-antibody-antigen/marker complex; and
c) detecting said antigen-antibody-antigen/marker complex

The immunoassay of the invention allows detecting antibodies specific to target antigens in a test sample. An antibody is an immunoglobulin produced by cells of the lymphocyte B series and secreted by plasma cells in response to stimulation by an antigen which specifically recognizes said antigen and is able to bind selectively to said antigen.

As it is used herein, the term "antigen" generally relates to any substance able to bind specifically to an antibody, or of inducing an immune response or of inducing the formation of antibodies, and it includes cells (e.g., tumor cells, etc.), organisms (e.g., viruses, bacteria, fungi, protozoa, nematodes, etc.), both complete and parts or components thereof (e.g., proteins, peptides, lipopolysaccharides, carbohydrates, etc.), of a native, synthetic or recombinant origin, which are identical or similar (i.e., able to be recognized by antibodies specific to native antigens) to the native antigens of an organism or of a cell, allergens, etc. virtually any antigen can be used in putting the immunoassay of the invention to detect antibodies specific to said antigen (target antigen) into practice.

Illustrative non-limiting examples of viral antigens include antigens of infectious and/or pathogenic viruses of animals, including human beings, such as viruses of the families: *Adenoviridae, Arenaviridae, Arteriviridae, Birnaviridae, Bungaviridae, Caliciviridae, Coronoviridae, Filoviridae, Flaviridae, Hepadnaviridae, Herpesviridae, Iridoviridae, Orthomyxoviridae*, *Papovaviridae, Paramyxoviridae, Parvoviridae, Picornaviridae*, *Poxviridae, Reoviridae, Retroviridae, Rhabdoviridae, Togaviridae,* etc., which include arbovirus, arterivirus, birnavirus, calicivirus, cytomegalovirus, herpesvirus, orbivirus, papillomavirus, parvovirus, pestivirus, poliovirus, retrovirus, rhinovirus, rotavirus, etc. Said viruses include pathogenic porcine viruses, for example, Porcine Parvovirus (PPV), Porcine Reproductive and , wherein the target antigen is an antigen of a virus belonging to the *Arteriviridae* or *Reoviridae* family of a virus belonging to the *Arteriviridae* or *Reoviridae* family Respiratory Syndrome Virus (PRRSV), Aujeszky's Disease Virus(ADV), Foot-and-mouth Disease Virus (FMDV), porcine Transmissible Gastroenteritis Virus (TGEV), Porcine Circovirus, etc.; bovine pathogens, such as Bovine Viral Diarrhea Virus (BVDV), Infectious Bovine Rhinotracheitis Virus (IBRV), Blue Tongue Virus (BTV), etc.; rabbit pathogens, such as Rabbit Hemorrhagic Disease Virus (RHDV), etc.; avian pathogens, for example, Infectious Bursal Disease Virus (IBDV), Avian Pneumovirus, etc. In a particular embodiment, said virus is Blue Tongue Virus (BTV) or Porcine Reproductive and Respiratory Syndrome Virus (PRRSV).

Illustrative non-limiting examples of bacterial antigens include antigens of pathogenic Gram positive and Gram negative bacteria of animals, including human beings. Specific examples of pathogenic bacteria include: *Actinornyces israelli, Bacillus aratracis, Bacteroides sp., Bordetella sp., Borelia burgdorferi, Brucella sp., Campylobacter sp.* (e.g., *C. jejuni*), *Clostridium sp.* (e.g., *C. perfringers, C. tetani), Corynebacterium sp.* (e.g., *C. diphtheriae*), *Enterobacter aerogenes, Enterococcus sp., Erysipelothrix rhusiopathiae, Escherichia coli, Fusobacterium nucleatum, Helicobacter pylori, Haemophilus influenzae, Klebsiella sp.* (e.g., *K. pneumoniae*), *Legionella pneumoplailia, Listeria monocytogenes, Leptospira, Moraxella catarrhalis, Mycobacteria sp.* (e.g., *M. tuberculosis, M. avium, M. intracellulare, M. kansaii, M. gordonae), Neisseria sp.* (e.g., *N. gonorrhoeae, N. meningitidis*), *Pasteurella sp.* (e.g., *P. multocida), Pseudomonas sp., Rickettsia sp., Salmonella sp., Staphylococcus sp.* (e.g., *S. aureus*), *Streptobacillus moniliformis, Streptococcus sp.* [e.g., *S. pyogefaes* (Group A Streptococcus), *S. agalactiae* (Group B Streptococcus), *S*. *viridans group, S. faecalis, S. bovis, Streptococcus* (anaerobic sp.), *S. pneumoniae*], *Treponema sp.* (e.g., *T. pallidum, T. pertenue*), etc.

Illustrative non-limiting examples of antigens of fungi include antigens of pathogenic fungi of animals, including human beings, such as *Blastomyces dermatitidis, Candida albicans, Chlamydia trachomatis, Coccidioides immitis, Cryptococcus neoformans, Histoplasma capsulatum,* etc.

Illustrative non-limiting examples of antigens of protozoa and nematodes include antigens of pathogenic protozoa and nematodes of animals, including human beings, such as *Anaplasma marginale, Dirofilaria immitis, Leishmania sp.* (leishmaniasis), *Plasmodium sp.* (malaria) [e.g., *P. falciparum, P. malariae, P. ovale, P. viva*x], *Schistosoma sp., Toxoplasma sp.* (e.g., *T. gondii*), etc.

Illustrative non-limiting examples of tumor antigens include molecules associated with a tumor or cancerous process that is able to cause an immune response when they are presented in the context of an MHC molecule. The selection of tumor antigens can be carried out by a person skilled in the art in view of the state of the art [Renkvist et al., Cancer Immunol. Immunother. 50:3-15 (2001)]. Representative non-limiting examples of said tumor antigens include: GD2 (neuroblastoma); EGF-R (malignant glioblastoma); CD52 (leukemia); human melanoma gp100 protein; human melanoma melan-A/MART-1 protein; tyrosinase; NA17-A nt protein; MAGE-3 protein; p53 protein; HPV16E7 protein; (CD11c)-TRP2; WT1 (leukemia and solid tumors); HM1.24 (multiple myeloma); prostate mucin (prostate adenocarcinoma); prostate-specific antigen (PSA); SF-25 antigen (colon adenocarcinoma); bladder tumor-associated antigens; MART-1 (melanoma); breast cancer-associated tumor antigens [e.g., Her2, mammaglobin, CA 15.3, CA 27.9, MCA (mucin-like carcinoma-associated antigen), MSA (mammary serum antigen), BMA (mucin antigen), cerbB2, MUC1, medullary thyroid cancer), etc.]; ovarian cancer-associated tumor antigens [e.g., CA125, SCC (immature teratoma), BHCG (choriocarcinoma), Her-2/neu, p21, etc]; lung cancer-associated tumor antigens [e.g., CD40, gp160, Cyfra-21.1, MAGE-A3, MUT-1, MUT-2, TPA, LAM8, SWA20, SCC (squamous cell carcinoma antigen), etc.]; skin cancer-associated tumor antigens [e.g., p97, gp75, EMA, MTA, MAGE-A1, MAGE-A3, etc.]; colon cancer-associated tumor antigens [e.g., COA-1, CA19.9, ND-4, CEA, TAG-72, CA72-4; B cell lymphoid tumor-associated tumor antigens [e.g., CD19, CD20, etc.]; myeloid leukemia-associated tumor antigens [e.g., CD33, etc.], tumor-associated antigens in lymphomas [e.g., CD 30, HSP70, etc.], etc., and antigenic fragments of said antigens. The tumor antigens can be prepared from tumor or cancerous cells by preparing crude extracts of said cells, for example, as described by Cohen, et al., in Cancer Research, 54:1055 (1994), by partially purifying the antigens by means of recombinant technology or by means of synthesis *de novo* of known antigens.

Other antigens are allergens, i.e., substances to which a subject is sensitive and causes an immune reaction, for example, allergenic extracts of pollens, allergenic extracts of insects, allergenic extracts of foods or of food products, components present in the saliva, pincers or stingers of insects which induce a sensitivity reaction in a subject, components present in plants which induce a sensitivity reaction in a subject, etc. Illustrative non-limiting examples of said allergens include allergenic extracts of grasses, arizonica species, platanaceae species, olive trees, etc. (including their pollens), allergenic extracts of insects (e.g., dust mites, etc.), allergenic extracts of foods or food products (e.g., gliadins, gluten, fish, shellfish, fruits, etc.). Virtually any allergen can be used in putting the immunoassay of the invention to detect antibodies specific to said allergen (target antigen) into practice.

The immunoassay of the invention can be used to detect antibodies specific to target antigens in a sample suspected of containing said antibodies (test sample), such as a biological sample, an environmental sample, a laboratory sample, etc. Illustrative non-limiting examples of biological samples include both tissue samples and bodily fluid samples that can contain antibodies specific to said target antigen (e.g., blood, serum, plasma, milk, saliva, sputum, cerebrospinal fluid, tissue exudates, etc.).

The test sample can be a sample of animal origin suspected of containing antibodies specific to target antigens. In a particular embodiment, said test sample is a sample from bovine, canine, caprine, equine, feline, leporine, sheep, porcine livestock etc., or a sample from a human being.

Before being contacted with the target antigen, the test sample can be subjected (or not) to a prior treatment, precipitated, fractionalized, separated, diluted, concentrated, or purified.

The test sample will generally be obtained through conventional methods known by persons skilled in the art, depending on the nature of the sample. In a particular embodiment, said test sample is a biological sample, such as a blood, serum or plasma sample, which can be obtained by any conventional method, for example, by means of a blood extraction, or a saliva sample, which can be obtained by means of using swabs, etc.

According to the immunoassay of the invention, the target antigen is contacted with said sample suspected of containing antibodies specific to said target antigen (i.e., able to specifically recognize or of selectively binding to said target antigen) under conditions allowing the formation of an antigen-antibody complex [step a)]. If the test sample contains antibodies specific to said target antigen, then said antigen-antibody complex will be formed; otherwise, said complex will not be formed. Therefore, according to the invention, said target antigen acts as a capture reagent for capturing antibodies specific to said target antigen. The suitable conditions so that the formation of the antigen-antibody complex can take place are known by persons skilled in the art.

A conjugate comprising said target antigen and a marker is subsequently added under conditions allowing the formation of an antigen-antibody-antigen/marker complex [step b)] and said antigen-antibody-antigen/marker complex is detected [step c)] If the test sample contains antibodies specific to said target antigen, an antigen-antibody complex will have been previously formed, thereby when said antigen-antibody complex is contacted with said conjugate comprising the target antigen and the marker , wherein the target antigen is an antigen of a virus belonging to the *Arteriviridae* or *Reoviridae* family in suitable conditions, the antigen-antibody-antigen/marker complex, which will be seen by means of the suitable technique depending on the marker used, as is mentioned below, is formed; whereas, otherwise, i.e., when the test sample does not contain antibodies specific to said target antigen, said antigen-antibody-antigen/marker complex will not be formed. Suitable conditions in order for the formation of the antigen-antibody-antigen/marker complex to take place are known by persons skilled in the art.

As it is used herein, the term "marker" relates to an indicator reagent which allows detecting the antigen-antibody-antigen/marker complex, such as an enzyme which catalyzes a detectable reaction, a compound which generates a signal when it forms part of the antigen-antibody-antigen/marker complex, etc. By way of non-limiting illustration, said marker can be an enzyme (e.g., peroxidase, glycosidase, alkaline phosphatase, glucose-6-phosphate dehydrogenase, β-galactosidase, β-glucosidase, β-glucuronidase, etc.), a fluorescent compound or fluorophore (e.g., fluorescein, rhodamine, etc.), a (chemo)luminescent compound (e.g., dioxetanes, acridiniums, phenanthridiniums, ruthenium, luminol, etc.), radioactive elements (sulfur, iodine, etc.), etc. In a particular embodiment, said marker is a peroxidase. The selection of a particular marker is not critical provided it is able to produce a signal by itself or together with one or more additional substances.

The conjugate comprising said target antigen and said marker can be obtained through conventional methods known by persons skilled in the art.

The antigen-antibody-antigen/marker complex formed can be detected or seen through by any suitable technique, depending on the chosen marker, known by persons skilled in the art, using suitable devices, for example, by means of techniques based on colorimetric, fluorometric, (chemo)luminescent, radioactive methods, etc., all known by persons skilled in the art.

By way of illustration, when the marker is an enzyme the detection of the antigen-antibody-antigen/marker complex can be carried out by contacting said complex with a suitable substrate, and optionally with suitable enzymatic amplification agents and/or activators. Illustrative examples of said substrates include:
- For alkaline phosphatase:
   Chromogenic: substrates based on p-nitrophenyl phosphate (p-NPP), 5-bromo-4-chloro-3-indolyl phosphate/nitroblue tetrazolium (BCIP/NBT), etc.
   Fluorogenic: 4-methylumbelliphenyl phosphate (4-MUP), 2-(5'-chloro-2'-phosphoryloxyphenyl)-6-chloro-4-(3H)-quinazolinone (CPPCQ), 3,6-fluorescein-diphosphate (3,6-FDP), etc.
- For peroxidases:
   Chromogenic: substrates based on 2,2-azinobis(3-ethylbenzthiazoline-6-sulfonic) (ABTS) acid, o-phenylenediamine (OPD), 3, 3', 5,5'-tetramethylbenzidine (TMB), o-dianisidine, 5-aminosalicylic acid, 3-dimethylaminobenzoic (DMAB) acid and 3-methyl-2-benzothiazolinehydrazone (MBTH), 3-amino-9-ethylcarbazole (AEC) and 3,3'-diaminobenzidine (DAB) tetrachloride, etc.
   Fluorogenic: 4-hydroxy-3-methoxyphenylacetic acid, reduced phenoxazines and reduced benzothiazines, including Amplex^{®} Red reagent, Amplex UltraRed reagent, reduced dihydroxanthens, etc.
- For glycosidases:
   Chromogenic: substrates based on o-nitrophenyl-β-D-galactoside (o-NPG), p-nitrophenyl-β-D-galactoside and 4-methylumbelliphenyl-β-D-galactoside (MUG) for β-D-galactosidase, etc.
   Fluorogenic: resorufin β-D-galactopyranoside, fluorescein digalactoside (FDG), fluorescein diglucuronide, 4-methylumbelliferyl beta-D-galactopyranoside, carboxyumbelliferyl beta-D-galactopyranoside, coumarin fluorinated beta-D-galactopyranosides, etc.

In a particular embodiment, said marker is a peroxidase, such as the horseradish peroxidase, and the chromogenic substrate is TMB.

The immunoassay of the invention can also be used to determine the amount of (to quantify) antibodies specific to a target antigen in a test sample since with many markers, e.g., enzymes, the amount of antibody present in the test sample is proportional to the signal generated.

The immunoassay of the invention is an immunoassay using labeled antigens to detect homogeneous or heterogeneous antibodies. In a particular embodiment, said immunoassay is an immunoassay using labeled heterogeneous antigens; illustrative non-limiting examples of such immunoassays include, depending on the marker used, enzyme immunoassays, such as ELISA (enzyme-linked immunosorbent assay), fluoroimmunoassays, such as DELFIA (dissociation-enhanced lanthanide fluoroimmunoassay), luminescent immunoassays, radioimmunoassays such as RIA (heterogeneous competitive radioimmunoassay), IRMA (heterogeneous non-competitive radioimmunoassay), etc. In a particular embodiment, said immunoassay is an ELISA.

To perform said heterogeneous immunoassays, the target antigen is directly or indirectly immobilized on a support, such as a well of a microtiter plate, magnetic beads, non-magnetic beads, columns, matrices, membranes, etc. These materials can be used in suitable forms, such as films, sheets, plates, etc., or it can be used to coat inert carriers (e.g., paper, glass, plastic films, etc.). The immobilization of the target antigen on said support includes ionic, hydrophobic, and/or similar interactions.

In a particular embodiment, the target antigen is directly or indirectly immobilized on a support, such as a well of a microtiter plate. The test sample suspected of containing antibodies specific to said target antigen is incubated with said target antigen for a time period and under conditions suitable in order for antigen-antibody complexes to be formed. After washing the well to remove the reagents not bound to the antigen, a conjugate comprising said target antigen bound to a marker is added and is left to incubate for a time period and under conditions suitable in order for antigen-antibody-antigen/marker complexes to be formed. After washing to remove the non-bound reagents, the formation of said antigen-antibody-antigen/marker complexes is developed. The detection of said antigen-antibody-antigen/marker complexes is indicative of the presence of antibodies specific to said target antigen in the test sample. This type of assay furthermore allows, if desired, quantifying the amount of antibodies specific to the target antigen in the test sample.

The formation of the antigen-antibody-antigen/marker complex is indicative of the presence of antibodies specific to the target antigen in the test sample. Therefore, the immunoassay of the invention can be used to diagnose the infection caused by an organism, such as a pathogenic organism (e.g., virus, bacterium, fungus, protozoon, nematode, etc.), in a subject that can be infected by said organism. As it is used herein, "subject" includes any animal, including human beings, in particular, vertebrate animals, preferably mammals, such as horses, pigs, rabbits, cats, sheep, dogs, cows, human beings, etc.

In a particular embodiment, the immunoassay of the invention can be used to diagnose the infection caused by BTV in animals that can be infected regardless of whether or not they develop clinical symptomatology (which can act as reservoirs), e.g., ovine, bovine, caprine, feline livestock, etc.

Therefore, in a specific embodiment, the immunoassay of the invention allows detecting and, if desired, quantifying specific antibodies against BTV in test samples from animals that can be infected by BTV by means of an ELISA (Example 1) which comprises contacting a BTV antigen immobilized on a support with a test sample suspected of containing antibodies specific to BTV; in the event that said test sample has said antibodies, these antibodies will bind to the BTV antigen. Then after removing the non-bound material by means of washings, again the same BTV antigen conjugated to a marker [antigen/marker conjugate] is added, and in the event that the test sample contains antibodies against said BTV antigen, said antibodies could capture the BTV antigen conjugated to the marker despite being bound to the BTV antigen immobilized on the well. The antigen-antibody-antigen/marker complex is detected after the addition of a suitable substrate.

Virtually any BTV antigen can be used in putting the previously described immunoassay into practice; nevertheless, by way of non-limiting illustration, in a particular embodiment said BTV antigen is a structural protein of BTV such as VP1, VP2, VP3, VP4, VP5, VP6, VP7, or a combination of two or more of said structural proteins; or a non-structural protein of BTV such as NS1, NS2, NS3, NS3a, or a combination of two or more of said non-structural proteins; or, alternatively, a mixture of one or more structural proteins of BTV with one or more non-structural proteins of BTV. Alternatively, antigenic fragments of said proteins can be used instead of whole proteins, provided that said fragments are able to be bound to antibodies against BTV, or fusion proteins comprising at least one of said proteins or one of said antigenic fragments thereof. Likewise, said proteins (and the antigenic fragments) can be both of a native and of a synthetic or recombinant origin. In a specific non-limiting embodiment, said BTV antigen is a recombinant VP7 protein of BTV (Example 1).

Virtually any of the supports referred to above in this description can be used as a support for putting the previously described immunoassay into practice; nevertheless, by way of non-limiting illustration, in a particular embodiment said support on which a BTV antigen is immobilized is a well of a microtiter plate.

Likewise, virtually any of the markers referred to above in this description, conjugated to a BTV antigen, can be used for putting the previously described immunoassay into practice; nevertheless, by way of non-limiting illustration, in a particular embodiment said marker is an enzyme, such as a peroxidase [BTV antigen/peroxidase conjugate], in which case the detection of the BTV antigen-antibody-BTV antigen/peroxidase complex is performed after the addition of a suitable substrate which develops color in the presence of the peroxidase (e.g., TMB).

In another particular embodiment, the immunoassay of the invention can be used to diagnose the infection caused by PRRSV in animals that can be infected by PRRSV regardless of whether or not they develop clinical symptomatology (reservoirs), e.g., porcine livestock, etc.

Therefore, in a specific embodiment the immunoassay of the invention allows detecting and, if desired, quantifying specific antibodies against PRRSV in test samples from animals that can be infected by BTV by means of an ELISA (Example 3) which comprises contacting a PRRSV antigen immobilized on a support with a test sample suspected of containing antibodies specific to PRRSV; in the event that said test sample has said antibodies, these antibodies will bind to the PRRSV antigen. Then after removing the non-bound material by means of washings, again the same PRRSV antigen conjugated to a marker [antigen/marker conjugate] is added, and in the event that the test sample contains antibodies against said PRRSV antigen, said antibodies could capture the PRRSV antigen conjugated to the marker despite being bound to the PRRSV antigen immobilized on the well. The antigen-antibody-antigen/marker complex is detected after the addition of a suitable substrate.

Virtually any PRRSV antigen can be used in putting the previously described immunoassay into practice; nevertheless, by way of non-limiting illustration, in a particular embodiment said PRRSV antigen is a structural or non-structural protein of PRRSV, or a combination of two, or more of said structural proteins, or a combination of two or more of said non-structural proteins, or alternatively a mixture of one or more structural proteins of PRRSV with one or more non-structural proteins of PRRSV. Alternatively, antigenic fragments of said proteins can be used instead of whole proteins, provided that said fragments are able to be bound to antibodies against PRRSV, or fusion proteins comprising at least one of said proteins or one of said antigenic fragments thereof. Likewise, said proteins (and the antigenic fragments) can be both of a native and of a synthetic or recombinant origin. In a specific non-limiting embodiment, said PRRSV antigen is a fusion protein comprising the nucleocapsid (N) protein of PRRSV (Example 3).

Virtually any of the supports referred to above in this description can be used as a support for putting the previously described immunoassay into practice; nevertheless, by way of non-limiting illustration, in a particular embodiment said support on which a PRRSV antigen is immobilized is a well of a microtiter plate.

Likewise, virtually any of the markers referred to above in this description, conjugated to a PRRSV antigen, can be used for putting the previously described immunoassay into practice; nevertheless, by way of non-limiting illustration, in a particular embodiment said marker is an enzyme, such as a peroxidase) [PRRSV antigen/peroxidase conjugate], in which case the detection of the PRRSV antigen-antibody-PRRSV antigen/peroxidase complex is performed after the addition of a suitable substrate which develops color in the presence of the peroxidase (e.g., TMB).

It is also disclosed an immunoassay that can be used to detect and, if desired, to quantify antibodies specific to tumor antigens in a test sample from a subject, and to thus diagnose the existence of a tumor or of a cancer in a subject, preferably in a human being.

It is also disclosed an immunoassay that can be used to detect and, if desired, to quantify antibodies specific to allergens in a test sample from a subject, and to thus diagnose the existence of an allergic reaction to said allergen in said subject, preferably in a human being.

If desired, positive and negative controls can furthermore be used for putting the immunoassay of the invention into practice.

The immunoassay of the invention is referred to as a "dual-recognition immunoassay" because it comprises the recognition and binding of two units of the target antigen to the specific antibody of said target antigen, one of them forming part of a conjugate comprising said target antigen and a marker.

The immunoassay of the invention has high specificity and sensitivity as is clearly shown in Example 2, in which it can be seen that the specificity of the immunoassay of the invention applied to the detection of antibodies specific to BTV is 99.8% and the sensitivity is 100%. This high sensitivity seems to be due to the fact that the dual-recognition immunoassay of the invention combines the two main advantages of indirect and competitive immunoassays because, on one hand, as occurs in indirect immunoassays, it is able to detect any type of antibody which binds to a target antigen (and not only the antibodies binding to a certain epitope, like in competitive assays), and, on the other hand, as in competitive (or blocking) assays, large amounts of sample (without diluting or scarcely diluted) can be used in the assay, which favors the detection of weak positives (in indirect assays the use dilutions of the sample of less than 1/100 is very rare). Likewise, the high specificity of the immunoassay of the invention seems to be due to the fact that the target antigen must be recognized two times by the same antibody, the first time when it is contacted with the target antigen immobilized on the solid phase or support used, and, the second time when it is contacted with the labeled target antigen (target antigen conjugate bound to a marker), typically in solution or without immobilizing.

Furthermore, if desired, positive and negative controls can be used for putting the immunoassay of the invention into practice.

In another aspect, the invention relates to a kit, hereinafter kit of the invention, comprising:
i) a support comprising a target antigen; and
ii) a conjugate comprising said target antigen and a marker .

The features of said support (i) and of the target antigen immobilized thereon, as well as the features of said conjugate (ii) have been mentioned above.

In a particular embodiment, the kit of the invention comprises, in addition to said support (i) and conjugate (ii), means for developing said marker.

Said kit of the invention is particularly useful for putting the immunoassay of the invention into practice.

Said target antigen can be recognized by an antibody specific thereto. Therefore, the kit of the invention can be used to detect, and if desired, to quantify antibodies specific to said target antigen in a sample suspected of containing said antibodies.

In a particular embodiment, the kit of the invention can be used to diagnose the infection caused by BTV in animals that can be infected by said virus (e.g., ovine, bovine, caprine, feline livestock, etc.) regardless of whether or not they present clinical symptomatology (in fact, a very interesting application of the kit and immunoassay of the invention consists of their use for identifying reservoirs of pathogenic organisms), and comprises a support comprising a BTV antigen and a conjugate comprising said BTV antigen conjugated to a marker, and optionally, means for developing the presence of the BTV antigen-antibody-BTV antigen/marker complex.

Virtually any BTV antigen can be used in said kit; nevertheless, by way of non-limiting illustration, in a particular embodiment said BTV antigen is a structural protein of BTV such as VP1, VP2, VP3, VP4, VP5, VP6, VP7, or a combination of two or more of said structural proteins; or a non-structural protein of BTV such as NS1, NS2, NS3, NS3a, or a combination of two or more of said non-structural proteins; or alternatively, a mixture of one or more structural proteins of BTV with one or more non-structural proteins of BTV. Alternatively, antigenic fragments of said proteins can be used , wherein the target antigen is an antigen of a virus belonging to the *Arteriviridae* or *Reoviridae* family instead of whole proteins, provided that said fragments are able to be bound to antibodies against BTV, or fusion proteins comprising at least one of said proteins or one of said antigenic fragments thereof. Likewise, said proteins (and the antigenic fragments) can be both of a native and of a synthetic or recombinant origin. In a specific non-limiting embodiment, said BTV antigen is a recombinant VP7 protein of BTV (Example 1).

The support present in said kit can be virtually any of the supports referred to above in this description; nevertheless, by way of non-limiting illustration, in a particular embodiment said support on which a BTV antigen is immobilized is a microtiter plate.

Likewise, virtually any of the markers referred to above in this description can be used in said conjugate comprising a BTV antigen and a marker; nevertheless, by way of non-limiting illustration, in a particular embodiment said marker is an enzyme, such as a peroxidase [BTV antigen/peroxidase conjugate], in which case said kit can contain, if desired, means for developing the presence of the BTV antigen-antibody-BTV antigen/marker complex (e.g., TMB when the marker is a peroxidase).

In another particular embodiment, the kit of the invention can be used to diagnose the infection caused by PRRSV in animals that can be infected by said virus (e.g., porcine livestock, etc.) regardless of whether or not they present clinical symptomatology, and comprises a support comprising a PRRSV antigen and a conjugate comprising said PRRSV antigen conjugated to a marker, and optionally means for developing the presence of the PRRSV antigen-antibody-PRRSV antigen/marker complex.

Virtually any PRRSV antigen can be used in said kit; nevertheless, by way of non-limiting illustration, in a particular embodiment said PRRSV antigen is a structural or non-structural protein of PRRSV, or a combination of two or more of said structural proteins, or a combination of two or more of said non-structural proteins, or alternatively, a mixture of one or more structural proteins of PRRSV with one or more non-structural proteins of PRRSV. Alternatively, antigenic fragments of said proteins can be used instead of whole proteins, provided that said fragments are able to be bound to antibodies against PRRSV, or fusion proteins comprising at least one of said proteins or one of said antigenic fragments thereof. Likewise, said proteins (and the antigenic fragments) can be both of a native and of a synthetic or recombinant origin. In a specific non-limiting embodiment, said PRRSV antigen is a fusion protein comprising the nucleocapsid (N) protein of PRRSV (Example 3).

The support present in said kit can be virtually any of the supports referred to above in this description; nevertheless, by way of non-limiting illustration, in a particular embodiment said support on which a PRRSV antigen is immobilized is a microtiter plate.

Likewise, virtually any of the markers referred to above in this description can be used in said conjugate comprising a PRRSV antigen and a marker; nevertheless, by way of non-limiting illustration, in a particular embodiment said marker is an enzyme, such as a peroxidase [PRRSV antigen/peroxidase conjugate], in which case said kit can contain, if desired, means for developing the presence of the PRRSV antigen-antibody-PRRSV antigen/marker complex (e.g., TMB when the marker is a peroxidase).

It is also disclosed a kit that can be used to detect and, if desired, to quantify antibodies specific to tumor antigens in a test sample from a subject, and to thus diagnose the existence of a tumor or of a cancer in a subject, preferably in a human being.

The following examples illustrate the invention and should not be considered as being limiting of the scope thereof.

### EXAMPLE 1

### Dual-recognition enzyme-linked immunosorbent assay for the detection of antibodies against Blue Tongue Virus (BTV)

For the purpose of validating this dual recognition enzyme-linked immunosorbent assay (DR ELISA) reference sera from different sources were analyzed for the 24 serotypes of BTV to detect specific antibodies against BTV.

### Materials and Methods

### rVP7 of BTV (BTV antigen)

The recombinant VP7 protein (rVP7) of BTV was used as the BTV antigen (Basak, A.K., Stuart D.I. and Roy P. 1992. J. Mol. Bio. 228:687-689). Said protein was obtained by infecting *Spodoptera frugiperda* Sf-9 cells with a recombinant baculovirus which contained the sequence encoding the VP7 protein of BTV and harvesting the culture with high cytopathic effect; after lysing the cells by means of osmotic shock, the supernatant obtained after the centrifugation was purified by ion exchange in FPLC (Fast Protein Liquid Chromatography) and the fractions which contained the protein were identified and titrated by means of sodium dodecyl sulfate polyacrylamide gel electrophoresis (SDS-PAGE) at 11% acrylamide. The rVP7 protein of BTV thus obtained and purified was used in coating the polystyrene plate at a concentration determined by ELISA in the range of 0.1-1 µg/ml.

### rVP7-HRPO conjugate

For its use as a conjugate, the rVP7 protein was dialyzed against 0.1 M bicarbonate buffer (pH 8.5) and conjugated with peroxidase (HRPO) (Horse Radish peroxidase, ROCHE) according to the method described by Nakane & Kawaoi [Nakane, P.K. & Kawaoi, A. (1974). Peroxidase-labeled antibody: A new method of conjugation. J. Histochem. Cytochem. 22: 1084-1091] modified herein. Each labeling process generated a conjugate production batch. Each of the production batches was stored diluted in phosphate buffered solution (PBS) pH 7.4 and 50% Surmodics-TM preserving solution (v/v) (Diarect AG) and was stored at +4°C until its use.

### Sera

For the purpose of validating this assay reference sera from different sources were analyzed for the 24 serotypes of BTV, said sources being:
- *Laboratorio de Referencia Español* (LRES) (Spanish Reference Laboratory) [Laboratorio Central de Veterinaria de Algete]: sera of serotypes 3, 10 and 19 of BTV;
- Reference Laboratory of the International Office of Epizootics (OIE) [Pirbright]: sera of the 24 serotypes of BTV; and
- Reference Laboratory of the United States Department of Agriculture [USDA] : sera of serotypes 5, 10, 19 and 20 of BTV.

### Controls

Control sera positive for BTV and control sera negative for BTV from animals immunized or not with VP7 of BTV, respectively, were used as controls.

### Dual recognition ELISA (DR ELISA)

96-well polystyrene plates are incubated with rVP7 protein of BTV for 18 hours at 4°C in carbonate-BSA (bovine serum albumin) buffer, pH 9.6, for the purpose of coating said plates with the BTV antigen (rVP7 protein). Then the plates are washed with PBS containing 0.05% Tween® 20, pH 7.4, are stabilized for 1 hour at room temperature (20-25°C) in Surmodics-TM (Diarect AG) stabilizing solution and are dried. The plates which are not going to be immediately used are individually packaged and stored at 4°C.

Then the sera to be analyzed are added on the plates coated with the BTV antigen at ½ dilution in PBS containing 0.05% Tween® 20 and are incubated for 1 hour at 37°C. After washing the non-adhered material with PBS containing 0.05% Tween® 20, the plates are incubated with an rVP7-HRPO conjugate at a predetermined dilution and titrated case by case for 1 hour at 37°C. After this time period has ended, the plates are washed with PBS containing 0.05% Tween® 20 and the reaction is developed for 15 minutes after the addition of the substrate TMB (tetramethylbenzidine). After stopping the reaction with 0.5 M sulfuric acid, absorbance is read at 450 nm in an ELISA plate reader.

### Results

The results obtained are shown in Table 1, where it can be seen that the assay provided by this invention (DR ELISA) is able to recognize all the serotypes of BTV for which there are reference sera in various Reference Laboratories (see the section relating to Sera). Likewise, said results clearly show that said DR ELISA is specific and sensitive enough to recognize all the serotypes of BTV, regardless of the type that they are and/or of the titer that they have.

**Table 1**

| Analysis of reference sera of 24 different serotypes of BTV | | | |
|---|---|---|---|
| **Serotype** | **DR ELISA** | | |
| | **LRES** | **Pirbright** | **USDA** |
| **1** | | + | |
| **2** | | + | |
| **3** | + | + | |
| **4** | | + | |
| **5** | | + | + |
| **6** | | + | |
| **7** | | + | |
| **8** | | + | |
| **9** | | + | |
| **10** | + | + | + |
| **11** | | + | |
| **12** | | + | |
| **13** | | + | |
| **14** | | + | |
| **15** | | + | |
| **16** | | + | |
| **17** | | + | |
| **18** | | + | |
| **19** | + | + | + |
| **20** | | + | + |
| **21** | | + | |
| **22** | | + | |
| **23** | | + | |
| **24** | | + | |

| | | | |
|---|---|---|---|
| [+: positive detection] | | | |

### EXAMPLE 2

### Specificity and sensitivity of the dual recognition enzyme-linked immunosorbent assay for the detection of antibodies against BTV using field sera

### 2.1 Specificity using field sera

To determine the specificity of the dual recognition enzyme-linked immunosorbent assay (DR ELISA) for the detection of antibodies against BTV, field studies were conducted with 758 sera from BTV-free farms. Cow and sheep sera were analyzed. The protocol followed was that described in Example 1 (DR ELISA). The results obtained showed a single positive sample with optical density (OD) values close to the cutoff point (established after determining the background signal of the negative field sera and observing the difference with the signal obtained by a borderline positive serum prepared in the laboratory, adjusting the assay so that said difference is at least 0.3 points of absorbance) indicating 99.8% specificity.

### 2.2 Sensitivity using field sera

To determine the sensitivity of the dual recognition enzyme-linked immunosorbent assay (DR ELISA) for the detection of antibodies against BTV, field studies were conducted with 758 sera from BTV-free farms. Following the protocol described in Example 1 (DR ELISA), a total of 288 cow and sheep sera previously characterized as positive by other techniques such as serum neutralization and other ELISA assays were analyzed. They were all positive, indicating 100% sensitivity.

### EXAMPLE 3

### Dual recognition enzyme-linked immunosorbent assay for the detection of antibodies against the Porcine Reproductive and Respiratory Syndrome Virus (PRRSV)

This dual recognition enzyme-linked immunosorbent assay (DR ELISA) was conducted to detect antibodies specific to PRRSV and to compare the results obtained with those of other already existing assays (indirect ELISA and blocking ELISA) for the detection of antibodies specific to PRRSV.

### Materials and Methods

### P10-N (PRRSV antigen)

The recombinant fusion protein identified as P10-N, comprising the amino acid sequence of the nucleocapsid of PRRSV, European isolate, fused to the amino acid sequence 1-259 of the protein of gene 10 of phage T7, was used as the PRRSV antigen, and the process for obtaining it is described in Example 3 of European patent EP 952219 B1.

### (P10-N)-HRPO conjugate

For its use as a conjugate, the P10-N fusion protein was conjugated with peroxidase (HRPO) according to the method described by Nakane & Kawaoi [Nakane, P.K. & Kawaoi, A. (1974). Peroxidase-labeled antibody: A new method of conjugation. J. Histochem. Cytochem. 22: 1084-1091] modified herein. Each labeling process generated a conjugate production batch. Each of the production batches was stored diluted in PBS pH 7.4 and preserving solution (Surmodics-TM, Diarect AG) 50% (v/v) and was stored at +4°C until its use.

### Sera

Thirty-six field sera from pigs of different geographical areas which were positive or negative in other assays (indirect ELISA and blocking ELISA) were analyzed for the detection of antibodies specific to PRRSV (Table 2).

### Indirect ELISA using the INGEZIM® PRRS EUROPA 1.1. PRS K1 (INGENASA) kit

100 µl of each of the test sera are added to the 1/100 dilution in the wells and are incubated for 1 hour at 37°C. Then the plate is washed 4 times with PBS containing 0.05% Tween® 20. Next, 100 µl of antibody specific to pig IgG conjugated with peroxidase are added at a predetermined dilution to each well, the plate is covered and incubated for 45 minutes at room temperature (20-25°C). The plate is subsequently washed 5 times with PBS containing 0.05% Tween® 20, 100 µl of ABTS substrate (2,2'-azino-bis(3-ethylbenzthiazoline-6-sulfonic acid) are added in each well and the reaction is maintained at room temperature for 15 minutes. Finally, 100 µl of 2% sodium dodecyl sulfate (SDS) are added to stop the reaction and absorbance is read at 405 nm in an ELISA reader.

### Blocking ELISA using the INGEZIM® PRRS COMPAC 1.1.PRS K3 kit (INGENASA)

The test sera are added at ½ dilution in the wells and are incubated for 1 hour at 37°C. Then the plate is washed 4 times with PBS containing 0.05% Tween® 20. Next, 100 µl of monoclonal antibody specific to the nucleocapsid (N) protein of PRRSV conjugated with peroxidase at a predetermined dilution are added and it is incubated for 20 minutes at 37°C. The plate is subsequently washed 5 times with PBS containing 0.05% Tween® 20, 100 µl of substrate ABTS are added in each well and the reaction is maintained for 15 minutes at room temperature. Finally, 100 µl of 2% SDS are added to stop the reaction and the absorbance is read at 405 nm in an ELISA reader.

### Dual recognition enzyme-linked immunosorbent assay (DR ELISA)

96-well polystyrene plates are incubated with P10-N fusion protein (PRRSV antigen) for 18 hours at 4°C in carbonate pH 9.6-BSA (bovine serum albumin) buffer for the purpose of coating said plates with the PRRSV antigen (P10-N fusion protein). Then the plates are washed with PBS containing 0.05% Tween® 20 pH 7.4, are stabilized for 1 hour at room temperature in stabilizing solution (Surmodics-TM, Diarect AG) and are dried. The plates which are not going to be immediately used are individually packaged and stored at 4°C.

Then the sera to be analyzed are added on the plates coated with the PRRSV antigen at 1/5 dilution in PBS containing 0.05% Tween® 20 in a volume of 50 µl and are incubated for 1 hour at 37°C. After washing the non-adhered material with PBS containing 0.05% Tween® 20, the plates are incubated with a (P10-N)-HRPO conjugate at a predetermined dilution and fixed case by case for 30 minutes at room temperature (20-25°C). After this time period has ended, the plates are washed with PBS containing 0.05% Tween® 20 and the reaction is developed for 15 minutes after the addition of the substrate TMB. After stopping the reaction with 0.5 M sulfuric acid, absorbance is read at 450 nm in an ELISA plate reader.

### Results

The results obtained are shown in Table 2, where it can be seen that the assay provided by this invention (DR ELISA) is able to detect antibodies specific to PRRSV and furthermore that said DR ELISA assay discriminates between positive and negative sera, comparing it with already existing competition and indirect immunoassays.

**Table 2**

| Comparison of the results obtained using the DR ELISA assay with the results obtained using an indirect ELISA and a blocking ELISA for the detection of antibodies specific to PRRSV | | |
|---|---|---|
| **Absorbance Values*** | | |
| **Dual recognition ELISA [DR ELISA]** | **INGEZIM® PRRS EUROPA 1.1.PRS K1 [indirect ELISA]** | **INGEZIM® PRRS COMPAC 1.1.PRS K3 [blocking ELISA]** |
| ***1.186*** | ***0.499*** | ***0.1715*** |
| ***1.86*** | ***0.8365*** | ***0.2045*** |
| ***3.071*** | ***1.4325*** | ***0**.**0965*** |
| 0.212 | 0.2225 | 1.0515 |
| ***0.437*** | ***0.37*** | 0.9945 |
| 0.214 | 0.151 | 1.1335 |
| ***1.423*** | ***0.7005*** | ***0.193*** |
| ***1.241*** | ***0.4605*** | 1.391 |
| 0.22 | ***0.566*** | 1.2915 |
| 0.212 | ***0.561*** | 1.19 |
| 0.205 | 0.1155 | 0.971 |
| ***0.411*** | 0.206 | 1.285 |
| 0.319 | 0.2295 | 1.133 |
| ***1.209*** | ***0.4545*** | ***0.817*** |
| ***0.474*** | ***0.3945*** | ***0.7885*** |
| 0.183 | 0.166 | 1.1675 |
| ***0.909*** | ***0.3875*** | ***0.666*** |
| ***0.74*** | ***0.4795*** | ***0.726*** |
| 0.206 | 0.102 | 1.13 |
| 0.263 | ***0.3665*** | 0.9715 |
| 0.342 | 0.2145 | 0.9855 |
| ***1.048*** | ***0.4905*** | ***0.69*** |
| ***0.692*** | 0.2455 | ***0.8435*** |
| ***1.095*** | ***0.2635*** | ***0.7035*** |
| ***1.046*** | ***0.355*** | 1.2675 |
| ***0.833*** | 0.1495 | 0.994 |
| ***0.706*** | ***0.2845*** | 0.885 |
| **1.777** | ***0.383*** | ***0.854*** |
| ***1.24*** | ***0.5405*** | 1.0265 |
| ***1.009*** | ***0.4415*** | ***0.8255*** |
| ***1.306*** | ***0.425*** | ***0.447*** |
| ***0.591*** | 0.223 | 1.18 |
| **Cut off:** | **Cut off:** | **Cut off:** |
| **0.3568** | **0.256725** | **0.8655** |

| | | |
|---|---|---|
| * Absorbance values obtained for a collection of 36 field sera. The sera considered positive for each of the assays are shown shaded, in bold print and in italics. | | |

## Claims

1. An immunoassay to detect an antibody specific to a target antigen in a sample which comprises:
a) contacting said target antigen with a sample suspected of containing said antibody specific to said target antigen under conditions allowing the formation of an antigen-antibody complex;
b) adding a conjugate comprising said target antigen and a marker under conditions allowing the formation of an antigen-antibody-antigen/marker complex; and
c) detecting said antigen-antibody-antigen/marker complex, wherein the target antigen is an antigen of a virus belonging to the *Arteriviridae* or *Reoviridae* family.

2. The immunoassay according to claim 1, wherein said virus is Porcine Reproductive and Respiratory Syndrome Virus (PRRSV) or Blue Tongue Virus (BTV).

3. The immunoassay according to claim 1, wherein said sample suspected of containing antibodies specific to said target antigen is a biological sample.

4. The immunoassay according to claim 1, wherein said marker is an enzyme, a fluorescent compound or fluorophore, a (chemo)luminescent compound or a radioactive element.

5. The immunoassay according to claim 4, wherein said marker is a peroxidase.

6. The immunoassay according to claim 1, wherein said immunoassay is an enzyme immunoassay, a fluoroimmunoassay, a luminescent immunoassay or a radioimmunoassay.

7. The immunoassay according to claim 6, wherein said immunoassay is an ELISA.

8. A kit comprising:
(i) a support comprising a target antigen; and
(ii) a conjugate comprising said target antigen and a marker,
wherein the target antigen is an antigen of a virus belonging to the *Arteriviridae* or *Reoviridae* family.

9. The kit according to claim 8, for diagnosing the infection caused by Blue Tongue Virus (BTV), comprising a support comprising a BTV antigen and a conjugate comprising said BTV antigen conjugated to a marker, and optionally means for developing the presence of BTV antigen-antibody-BTV antigen/marker complex.

10. The kit according to claim 9, wherein said BTV antigen is a recombinant VP7 protein of BTV (rVP7) and said conjugate comprises said rVP7 protein of BTV conjugated to a peroxidase, and it furthermore comprises 3,3',5,5'-tetramethylbenzidine (TMB).

11. The kit according to claim 8, for diagnosing the infection caused by Porcine Reproductive and Respiratory Syndrome Virus (PRRSV) comprising a support comprising a PRRSV antigen and a conjugate comprising said PRRSV antigen conjugated to a marker, and optionally means for developing the presence of PRRSV antigen-antibody-PRRSV antigen/marker complex.

12. The kit according to claim 11, wherein said PRRSV antigen is a fusion protein comprising the nucleocapsid (N) protein of PRRSV and said conjugate comprises said fusion protein comprising the protein N of PRRSV conjugated to a peroxidase, and it furthermore comprises 3,3',5,5'-tetramethylbenzidine (TMB).

## Patentansprüche

1. Ein Immuntest für das Detektieren eines Antikörpers, der spezifisch zu einem Zielantigen ist, in einer Probe, welcher umfasst:
a) Inkontaktbringen des Zielantigens mit einer Probe, von der vermutet wird, dass diese die Antikörper, welche zu dem Zielantigen spezifisch sind, zu enthalten, unter Bedingungen, die das Bilden eines Antigen-Antikörper-Komplexes erlauben;
b) Hinzufügen eines Konjugats, welches das Zielantigen und einen Marker umfasst, unter Bedingungen, die das Bilden eines Antigen-Antikörper-Antigen/Marker-Komplexes erlauben; und
c) Detektieren des Antigen-Antikörper-Antigen/Marker-Komplexes,
wobei das Zielantigen ein Antigen eines Virus aus der Familie der *Arteriviridae* oder *Reoviridae* ist.

2. Immuntest gemäß Anspruch 1, wobei das Virus *Porcine Reproductive and Respiratory Syndrome*-Virus (PRRSV) oder Blauzungenvirus (BTV) ist.

3. Immuntest gemäß Anspruch 1, wobei die Probe, von der vermutet wird, Antikörper, die zu dem Zielantigen spezifisch sind, zu enthalten, eine biologische Probe ist.

4. Immuntest gemäß Anspruch 1, wobei der Marker ein Enzym, eine fluoreszierende Verbindung oder ein Fluorophor, eine (chemo-)lumineszierende Verbindung oder ein radioaktives Element ist.

5. Immuntest gemäß Anspruch 4, wobei der Marker eine Peroxidase ist.

6. Immuntest gemäß Anspruch 1, wobei der Immuntest ein Enzym-Immuntest, ein Fluoro-Immuntest, ein Lumineszenz-Immuntest oder ein Radio-Immuntest ist.

7. Immuntest gemäß Anspruch 6, wobei der Immuntest ein ELISA ist.

8. Kit, welcher umfasst:
(i) einen Träger, welcher ein Zielantigen umfasst; und
(ii) ein Konjugat, welches das Zielantigen und einen Marker umfasst,
wobei das Zielantigen ein Antigen eines Virus aus der Familie der *Arteriviridae* oder *Reoviridae* ist.

9. Kit gemäß Anspruch 8, für die Diagnose der Infektion, die durch Blauzungenvirus (BTV) verursacht wird, umfassend eines Trägers, welcher ein BTV-Antigen umfasst, und eines Konjugats, welches das BTV-Antigen, das an einen Marker konjugiert ist, umfasst, und gegebenenfalls Mittel zum Entwickeln der Anwesenheit des BTV-Antigen-Antikörper-BTV-Antigen/Marker-Komplexes.

10. Kit gemäß Anspruch 9, wobei das BTV-Antigen ein rekombinantes Protein von VP7 BTV (rVP7) ist und das Konjugat das rVP7 Protein von BTV, welches an eine Peroxidase konjugiert ist, umfasst, und das ferner 3,3',5,5'-Tetramethylbenzidin (TMB) umfasst.

11. Kit gemäß Anspruch 8, für die Diagnose der Infektion, die durch *Porcine Reproductive and Respiratory Syndrome-Virus* (PRRSV) verursacht wird, umfassend eines Trägers, welcher ein PRRSV-Antigen umfasst, und eines Konjugats, welches das PRRSV-Antigen, das an einen Marker konjugiert ist, umfasst, und gegebenenfalls Mittel zum Entwickeln der Anwesenheit des PRRSV-Antigen-Antikörper-PRRSV-Antigen/Marker-Komplexes.

12. Kit gemäß Anspruch 11, wobei das PRRSV-Antigen ein Fusionsprotein ist, das das Nukleokapsidprotein (Protein N) von PRRSV umfasst, und das Konjugat das Fusionsprotein, das das Protein N von PPRSV umfasst, welches an eine Peroxidase konjugiert ist, umfasst, und das ferner 3,3',5,5'-Tetramethylbenzidin (TMB) umfasst.

## Revendications

1. Dosage immunologique permettant de détecter un anticorps spécifique d'un antigène cible dans un échantillon comprenant :
a) mettre en contact ledit antigène cible avec un échantillon suspecté de contenir ledit anticorps spécifique dudit antigène cible dans des conditions permettant la formation d'un complexe antigène-anticorps;
b) ajouter un conjugué contenant ledit antigène cible et un marqueur dans des conditions permettant la formation d'un complexe antigène-anticorps-antigène/marqueur ; et
c) détecter ledit complexe antigène-anticorps-antigène/marqueur,
dans lequel l'antigène cible est un antigène d'un virus appartenant à la famille des *Arteriviridae* ou *Reoviridae.*

2. Dosage immunologique selon la revendication 1, dans lequel ledit virus est le Virus du Syndrome Reproducteur et Respiratoire Porcin (VSRRP) ou le Virus de la Fièvre Catarrhale (VFC).

3. Dosage immunologique selon la revendication 1, dans lequel ledit échantillon suspecté de contenir des anticorps spéciques dudit antigène cible est un échantillon biologique.

4. Dosage immunologique selon la revendication 1, dans lequel ledit marqueur est une enzyme, un composé fluorescent ou un fluorophore, un composé (chimio)luminescent ou un élément radioactif.

5. Dosage immunologique selon la revendication 4, dans lequel ledit marqueur est une peroxydase.

6. Dosage immunologique selon la revendication 1, dans lequel ledit dosage immunologique est un dosage enzymo-immunologique, un dosage fluoro-immunologique, un dosage lumino-immunologique ou un dosage radio-immunologique.

7. Dosage immunologique selon la revendication 6, dans lequel ledit dosage immunologique est un dosage enzymo-immunologique en phase solide (ELISA).

8. Kit comprenant :
(i) un support comprenant un antigène cible ; et
(ii) un conjugué comprenant ledit antigène cible et un marqueur,
dans lequel l'antigène cible est un antigène d'un virus appartenant à la famille des *Arteriviridae* ou *Reoviridae.*

9. Kit selon la revendication 8, pour diagnostiquer l'infection provoquée par le virus de la fièvre catarrhale (VFC) comprenant un support comprenant un antigène VFC et un conjugué comprenant ledit antigène VFC conjugué à un marqueur, et éventuellement des moyens permettant de développer la présence du complexe antigène VFC-anticorps-antigène VFC/marqueur.

10. Kit selon la revendication 9, dans lequel ledit antigène VFC est une protéine recombinante VP7 du VFC (rVP7) et ledit conjugué comprend ladite protéine rVP7 du VFC conjuguée à une peroxydase, et comprend en outre du 3,3',5,5'-tétraméthylbenzidine (TMB).

11. Kit selon la revendication 8, pour diagnostiquer l'infection provoquée par le Virus du Syndrome Reproducteur et Respiratoire Porcin (VSRRP) comprenant un support comprenant un antigène VSRRP et un conjugué comprenant ledit antigène VSRRP conjugué à un marqueur, et éventuellement des moyens permettant de développer la présence du complexe antigène VSRRP-anticorps-antigène VSRRP/marqueur.

12. Kit selon la revendication 11, dans lequel ledit antigène VSRRP est une protéine de fusion comprenant la protéine de nucléocapside (N) du VSRRP et ledit conjugué comprend ladite protéine de fusion comprenant la protéine N du VSRRP conjuguée à une peroxydase, et comprend en outre du 3,3',5,5'-tétraméthylbenzidine (TMB).
